Europäisches Patentamt

European Patent Office

Office européen des brevets

(11) Publication number: **0 473 158 A1**

# EUROPEAN PATENT APPLICATION

(21) Application number: **91114548.0**

(22) Date of filing: **29.08.91**

(51) Int. Cl.⁵: **C12Q 1/68**

(30) Priority: **30.08.90 JP 226769/90**

(43) Date of publication of application:
**04.03.92 Bulletin 92/10**

(84) Designated Contracting States:
**DE FR GB IT**

(71) Applicant: **Tosoh Corporation**
**4560, Kaisei-cho**
**Shinnanyo-shi, Yamaguchi-ken 746(JP)**

(72) Inventor: **Hara, Tadashi**
**2398, Kawaraguchi**
**Ebina-shi, Kanagawa-ken(JP)**
Inventor: **Inouye, Kuniyo**
**5-jyo-agaru, Sakaimachi-tori**
**Shimogyo-ku, Kyoto-shi, Kyoto(JP)**

(74) Representative: **Vossius & Partner**
**Siebertstrasse 4 P.O. Box 86 07 67**
**W-8000 München 86(DE)**

(54) **Method for the immunoassay of a nucleic acid.**

㊳ The quantity of a nucleic acid in a sample is determined by an immunoassay method wherein (a) an immobilized first monoclonal antibody recognizing a specific base sequence, (b) a single-stranded nucleic acid, (c) a nucleic acid in a sample, which has been rendered single-stranded, and (d) a second monoclonal antibody structure-specifically recognizing a double-stranded nucleic acid are contacted with each other to effect an immunoreaction. Either the nucleic acid probe (b) or the nucleic acid (c) has been linked with the specific base sequence before the reaction. The free nucleic acid, free antibodies and free nucleic acid probe are removed from the immunoreaction mixture, and then, the quantity of the thus-produced immunoreaction product is determined.

EP 0 473 158 A1

EP 0 473 158 A1

This invention relates to the immunoassay of a nucleic acid in a sample. More particularly, it relates to a method for the immunoassay of a nucleic acid in a sample wherein either a single-stranded nucleic acid in a sample or a single-stranded nucleic acid probe having a specific base sequence linked thereto is immobilized to a solid phase with a first monoclonal antibody specifically recognizing this specific base sequence, and a second monoclonal antibody specifically recognizing a double-stranded nucleic acid is utilized.

The method of identifying a base sequence of a nucleic acid is a diagnosis method attracting great attention at present, and it is expected that this diagnosis method will be applied to the diagnosis of infectious diseases, the diagnosis of gene diseases and the diagnosis of cancers. For example, the method will be applied to the diagnosis of infectious diseases caused by bacteria, such as Salmonella and Shigella, hepatitis and AIDS caused by viruses, and infectious diseases caused by microorganisms such as mycoplasmas, parasites and Chlamydia.

In the field of the diagnosis of gene diseases, it is expected that the method is applied to the clinical diagnosis of diseases caused by gene deficiency, for example, chromosomal hereditary diseases such as Down's syndrome and Turner's syndrome, Mendelian hereditary diseases such as phenylketonuria and hemophilia, and multiple-factor-type hereditary diseases such as essential hypertension and diabetes. Furthermore, in the case of cancers, the diagnosis can be performed by identifying cancer genes generated by mutation or activation of normal DNA sequences. Namely, it is expected that if a proper DNA probe is used, the clinical diagnosis can be performed by specifically identifying a gene or a part thereof, concerned with the disease.

As the method of determination a DNA, there are generally adopted four assays, described below, utilizing hybridization of nucleic acids. Namely, there are adopted the dot hybridization method [P. Stalhandske and U. Pettersson, J. Clin. Microbiol., 15, 744-747 (1982)], the colony hybridization method [M. J. Haas et al, Pers. Commun. (1987)], the in situ hybridization method [C. J. Burrell, E. J. Gowans, A. R. Jilbert, J. R. Lake and B. P. Marmion, Hepatol., 2, 85s-91s (1982)], and the sandwich hybridization method [M. Virtanen, M. Laaksonen, H. S. Derlund, A. Palva, P. Halonen and M. Ranki, Lancet., 1, 381-389 (1983)].

The dot hybridization method will now be described. At first, a nucleic acid in a sample is extracted and subjected to an alkali treatment, e.g., with 0.5N NaOH, to convert it to a single-stranded DNA. This single-stranded DNA is immobilized on a nitrocellulose filter and is then hybridized by addition of a radioactively labeled hybridization probe. The free hybridization probe is removed and the radioactively labeling substance is detected.

Alternatively, as a method using a non-radioactive hybridization probe, there is commercially used a method in which a nucleic acid is labeled with a hapten and the labeled nucleic acid is immunologically detected [J. E. Landegent et al, Exp. Cell. Res., 153, 61-72 (1984), and P. Tchen et al, Proc. Natl. Acad. Sci. U.S.A., 81, 3466-3470 (1984)], a method in which a biotin-labeled DNA probe is hybridized with DNA in a sample and coloration is effected by streptavidin, alkaline phosphatase and NBT coloring matter [Toyozo Takahashi and Kenji Okuda, Bioindustry, 2, 928-935 and 1013-1015 (1985)], a photobiotin method developed by BRESA (Biotechnology Research Enterprises South Australia Pty. Ltd.), Australia, a chemiprobe method utilizing an antibody developed by Orgenics Co., Ltd., and a method in which a target DNA is labeled with labezyme-peroxidase developed by Wako Junyaku K.K. and the labeled DNA is detected.

Among patent references, Japanese Unexamined Patent Publication No. 60-72828 is important.

The known method using a radioactively labeled hybridization probe has an advantage in that many samples can be detected very simply. However, since labeling is effected with a radioactive substance, the method becomes economically disadvantageous, and the safety to human bodies, the safety of the discharge treatment of wastes, the arangement of a proper equipment for the use of radioactive substances should be taken into consideration. The method using a non-radioactively labeled hybridization probe has problems such that the sensitivity is low, and since a filter is used, a particular contrivance should be made to the washing method.

The primary object of the present invention is to solve the problems of the conventional techniques and provide a method of the immunological diagnosis of nucleic acids, which is characterized by a simple operation, low costs, a high safety and a high sensitivity. This object can be achieved by using a monoclonal antibody which is structurally specific to a nuleic acid.

More specifically, in one aspect of the present invention, there is provided a method for the immunoassay of a nucleic acid, which comprises the steps of:

contacting with each other (a) an immobilized first monoclonal antibody recognizing a specific base sequence, (b) a single-stranded nucleic acid probe having the specific base sequence linked to the 5'-terminal or 3'-terminal, (c) a nucleic acid in a sample which has been rendered single-stranded, and (d) a second monoclonal antibody structure-specifically recognizing a double-stranded nucleic acid;

2

removing the free nucleic acid, free antibodies and free nucleic acid probe from the immunoreaction mixture; and then,

determining the quantity of the thus-produced immunoreaction product.

In another aspect of the present inventon, there is provided a method of the immunoassay of a nucleic acid, which comprises the steps of:

contacting with each other (a) an immobilized first monoclonal antibody recognizing a specific base sequence, (b) a nucleic acid in a sample which has been rendered single-stranded and linked to the specific base sequence, (c) a single-stranded nucleic acid probe, and (d) a second monoclonal antibody structure-specifically recognizing a double-stranded nucleic acid;

removing the free nucleic acid, free antibodies and free nucleic acid probe from the immunoreaction mixture; and then,

determining the quantity of the thus-produced immunoreaction product.

The two methods of the present invention will now be described in detail.

The nucleic acid in a sample to be assayed by the methods of the present invetion is not particularly limited, and there can be mentioned single-stranded and double-stranded nucleic acids derived from animals and plants, and naturally occurring microorganisms such as viruses and bacteria. In the case of a double-stranded nucleic acid, the reaction of the present invention is carried out after melting of the double-stranded nucleic acid to a single-stranded nucleic acid.

The specific base sequence referred to in the present invention means a sequence comprising a plurality of the same bases connected in series, and preferably at least 10 bases connected in series. For example, there can be mentioned poly-dT, poly-dA, poly-dC and poly-dG. The base sequence is essential for the recognition by a monoclonal antibody.

In the first method of the present invention, a single-stranded nucleic acid probe having the specific base sequence linked to the 5'-terminal or the 3'-terminal of the nucleic acid prove is used. The base sequence may be linked to either the 5'-terminal or the 3'-terminal, and the linking method is not particularly limited and any known method can be adopted. In the second method of the present invention, a nucleic acid in a sample is rendered single-stranded and linked with the specific base sequence,and thereafter used for the immunoassay.

Either DNA or RNA can be used as the nucleic acid in the present invention.

The first monoclonal antibody recognizing a specific base sequence and the second monoclonal antibody structure-specifically recognizing a double-stranded nucleic acid can be prepared according to the method of G. Köhler and C. Milstein (Nature, 256, 495, 1975). Commercially available monoclonal antibodies can also be used.

Known physical and chemical methods can be used for immobilizing the first monoclonal antibody to a solid phase. Alternatively, a method can be adopted in which a monoclonal or polyclonal antibody specifically recognizing the Fc region of the first monoclonal antibody is physically or chemically immobilized to a solid phase, and then, the first monoclonal antibody is immobilized on the above-mentioned immobilized monoclonal or polyclonal antibody.

As the solid phase, there can be mentioned, for example, polystyrene, polyethylene, polyvinyl chloride, polycarbonate, Sepharose particles, latex, agarose, cellulose and polymethacrylates.

By labeling the second monoclonal antibody, the finally produced immunoreaction product can be detected easily and simply. This labeling method and this detecting method are not particularly limited, and labeling and detection can be carried out by known methods. As the label, there can be used radioactive substances such as $^3$H, $^{125}$I and $^{131}$I, but in view of the safety and ease of handling, a fluorescent substance such as fluorescamine, fluorescein isothiocyanate and tetrarhodamine isothiocyanate are preferably used for the labeling of the second monoclonal antibody. More preferably, there can be used enzymes used in the ordinary enzyme immunoassay, such as peroxidase, $\beta$-D-galactosidase, alkaline phosphatase, urease, catalase and $\beta$-glucuronidase.

When an unlabeled second monoclonal antibody is used, an immunoreaction complex having a labeling substance is produced on a solid phase by using a monoclonal or polyclonal antibody specifically recognizing the Fc region of the second monoclonal antibody and labeled with a labeling substance as mentioned above.

Thus, in the first method of the present invention, an immunoreaction complex represented by the following formula is produced:

solid phase··first monoclonal antibody--single-stranded nucleic acid probe having linked thereto a specific base sequence--nucleic acid in sample--second monoclonal antibody··labeling substance.

In this formula, "···" means a linkage by which each of the first monoclonal antibody and the second monoclonal antibody may be connected either directly or through a monoclonal or polychlonal antibody.

The connection of the second monoclonal antibody to a solid phase through a monoclonal or polyclonal antibody is effected as follows. A monoclonal or polyclonal antibody specifically recognizing the Fc region of the second monoclonal antibody is chemically or physically immobilized to a solid phase, and then, the second monoclonal antibody is immobilized on the abovementioned immobilized monoclonal or polyclonal antibody.

In the second method of the present invention, an immunoreaction complex is produced which is analogous to the above complex, but is different in that the single-stranded nucleic acid probe having linked thereto a specific base sequence and the nucleic acid in sample are substituted by "single-stranded nucleic acid in sample having linked thereto a specific base sequence" and "single-stranded nucleic acid probe", respectively.

When the first monoclonal antibody and the second monoclonal antibody used belong to the same subclass and thus have the same Fc region, it is apprehended that the labeled monoclonal or polyclonal antibody prepared so as to recognize the Fc region of the second monoclonal antibody will erroneously recognize the Fc region of the first monoclonal antibody to produce a complex represented by the formula: "solid phase‥first monoclonal antibody ‥labeling substance". Accordingly, in the present invention, to simplify operations such as preparation of antibodies, it is preferable to use two kinds of monoclonal antibodies, that is, a first monoclonal antibody physically or chemically linked to the solid phase and a second monoclonal antibody chemically linked to the labeling substance.

It is preferable that the first monoclonal antibody specifically recognizing the specific base sequence of a single-stranded nucleic acid and the second monoclonal antibody specifically recognizing a double-stranded nucleic acid have different sub-classes, and the first and second monoclonal antibodies are contacted with a labeled monoclonal or polyclonal antibody recognizing the Fc region of the second monoclonal. It is also preferable to use a second monoclonal antibody linked directly, i.e., without the use of an intervening monoclonal or polyclonal antibody, to a labeling substance.

In the hybridization and immunological reaction, the order of contact of the first monoclonal antibody, the nucleic acid probe, the nucleic acid in the sample and the second monoclonal antibody is not particularly limited. These ingredients can be contacted in sequence and unreacted ingredients can be removed during the reaction according to the need. Alternatively, all of these ingredients are contacted with one another at a time to effect the reaction.

In each case, finally, among the labeled second monoclonal antibodies, the free (i.e., not linked indirectly to the solid phase) labeled second monoclonal antibody can be removed by a washing method customarily adopted in the immunoassay, for example, the B/F separation method.

According to the present invention, nucleic acids can be assayed by simple operations. Furthermore, by carrying out in advance immobilization of a first monoclonal antibody and linking of a labeling substance to a second monoclonal antibody, the immunoassay operations can be simplified and shortened. This means that a clinical desire to measure many samples in a short time can be satisfied.

Moreover, in the present invention, since a first monoclonal antibody recognizing a specific base sequence and a second monoclonal antibody structure-specifically recognizing a double-stranded nucleic acid are used, linking of the monoclonal antibodies does not hinder the hybridization of nucleic acid.

The present invention will now be described in detail with reference to the following examples that by no means limit the scope of the invention.


(1) Immobilization of Poly-dT Monoclonal Antibody


To each well of an untreated microtiter plate (Nunc plate composed of 96 wells, supplied by Intermed Co.) were added 100 $\mu$l of a solution prepared by dissolving a poly-dT (pdT) monoclonal antibody (supplied by Chemicon Co.) at a concentration of 10 $\mu$g/ml in a 0.1M carbonate buffer solution (pH 9.6), and incubation was carried out at room temperature for 2 hours. The carbonate buffer solution was removed from each well, and washing was conducted three times with phosphate-buffered physiological saline solution (0.01% by weight phosphate buffer solution containing 0.85% by weight of NaCl, pH = 7.2; hereinafter referred to as "PBS"). Then 300 $\mu$l of a solution of 0.1% by weight bovine serum albumin (hereinafter referred to as "BSA") in PBS was added to each well and the blocking treatment was carried out at 4°C.

The plate having the monoclonal antibody immobilized thereon was preserved at 4°C until the measurement.


(2) Measurement of Nucleic Acid in Sample

The temperature of the plate prepared in (1) was returned to room temperature and the plate was washed with a phosphate-buffered physiological saline solution containing 0.04% by weight of Tween-20 (hereinafter referred to as "PBS-T"). Then 100 $\mu$l of a 10 $\mu$g/ml nucleic acid probe having pdT (composed of at least 10 T's, supplied by Sigma Co.) linked through ligase to the terminals was added to each well, and incubation was carried out at 37°C for 1.5 hours.

(3) Preparation of Single-Stranded Nucleic Acid

A 0.18M NaCl solution containing a predetermined amount of a double-stranded nucleic acid was boiled for 5 minutes. Then the solution was immediately cooled with ice to prepare a solution of a single-stranded nucleic acid.

(4) Hybridization

The single stranded nucleic acid solution prepared in (3) was dissolved in a hybridization solution [final concentration of 5 x SSC, 1 x Denhalt solution, 5% by weight dextransulfate, 20 mM phosphate buffer solution (pH = 6.5)], and 100 $\mu$l of the solution was added to each well and incubation was carried out at 65°C overnight. Then washing was carried out with PBS-T three times.

(5) Measurement of Single-Stranded Nucleic Acid in Sample

A mouse double-stranded nucleic acid monoclonal antibody was diluted with PBS-T solution, and 100 $\mu$l of the diluted solution was added to each well. Incubation was carried out at 37°C for 1.5 hours, and then, the solution was removed and washing was carried out three times with PBS-T solution. Then 100 $\mu$l of a peroxidase-labeled immunogloblin antibody (supplied by Tago Co.) diluted with PBS-T solution was added to each well, and incubation was carried out at 37°C for 1.5 hours. Then the solution was removed and washing was carried out three times with PBS-T solution. Then 100 $\mu$l of a substrate solution comprising a 0.1M citrate buffer solution (pH = 4.1) containing 0.03% by weight of 2,2-aminodi-(3-ethylbenz-thiazoline sulfate)-diammonium salt (ABTS) and 0.01% by weight of hydrogen peroxide ($H_2O_2$) was added to each well and the enzyme reaction was left to take place at room temperature for ten minutes. Then 100 $\mu$l of a 200 mM oxalic acid solution was added to stop the enzyme reaction.

With regard to each well of the microtiter plate, the absorbance intensity was measured at a wavelength of 415 nm and a reference wavelength of 492 nm by an automatic microtiter plate reader ("MPR-A4" tradename, supplied by Tosoh Corp.). The results are shown in the following table. As seen from the results shown in Table 1, it was confirmed that the nucleic acid in the sample could be determined in the concentration range of from 0.1 to 100 ng/ml.

Table 1

| Nucleic acid concentration (ng/ml) | Absorbance intensity |
|---|---|
| 0.1 | 0.05 |
| 0.3 | 0.1 |
| 1 | 0.17 |
| 3 | 0.23 |
| 10 | 0.32 |
| 30 | 0.4 |
| 100 | 0.49 |

**Claims**

1. A method for the immunoassay of a nucleic acid, which comprises the steps of:
   contacting with each other
   (a) an immobilized first monoclonal antibody recognizing a specific base sequence,
   (b) a single-stranded nucleic acid probe having the specific base sequence linked to the 5'-terminal or the 3'-terminal,
   (c) a nucleic acid in a sample which has been rendered single-stranded, and

(d) a second monoclonal antibody structure-specifically recognizing a double-stranded nucleic acid;
removing the free nucleic acid, free antibodies and free nucleic acid probe from the immunoreaction mixture; and then,
determining the quantity of the thus-produced immunoreaction product.

2. A method of the immunoassay of a nuceic acid, which comprises the steps of:
contacting with each other
(a) an immobilized first monoclonal antibody recognizing a specific base sequence,
(b) a nucleic acid in a sample which has been rendered single-stranded and linked to the specific base sequence,
(c) a single-stranded nucleic acid probe, and
(d) a second monoclonal antibody structure-specifically recognizing a double-stranded nucleic acid;
removing the free nucleic acid, free antibodies and free nucleic acid probe from the immunoreaction mixture; and then,
determining the quantity of the thus-produced immunoreaction product.

3. The method according to claim 1 or 2, wherein the specific base sequence consists of at least 10 bases, which are the same and connected in series.

4. The method according to any of claims 1 through 3, wherein said first monoclonal antibody is immobilized on a solid phase either directly or through a monoclonal or polyclonal antibody specifically recognizing the Fc region of the first monoclonal antibody.

5. The method according to any of claims 1 through 4, wherein (1) the second monoclonal antibody is labeled with a fluorescent substance or an enzyme; or (2) the second monoclonal antibody is not labeled, and a monoclonal or polyclonal antibody specifically recognizing the Fc region of the second monoclonal antibody and labeled with a fluorescent substance or an enzyme is further incorporated.

6. The method according to any of claims 1 through 5, wherein the first monoclonal antibody is physically or chemically immobilized to a solid phase and the second monoclonal antibody is chemically linked to a fluorescent substance or an enzyme.

7. The method according to any of claims 1 through 3, wherein the first monoclonal antibody and the second monoclonal antibody are of different sub-classes, and the first and second monoclonal antibodies are contacted with a labeled monoclonal or polyclonal antibody recognizing the Fc region of the second monoclonal antibody.

European
Patent Office

EUROPEAN SEARCH
REPORT

Application Number

EP 91 11 4548

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 135 159   (CETUS CORPORATION) <br> * page 6, line 18 - page 7, line 5 * * * page 8, line 3 - line 28; claims * * <br> – – – | 1 | C 12 Q 1/68 |
| A | EP-A-0 163 220   (MILES LABORATORIES) <br> * page 17, line 22 - page 24, line 16 * * <br> – – – | 1 | |
| A | EP-A-0 204 510   (AMOCO CORPORATION) <br> * column 10, line 21 - line 43 * * <br> – – – | 1 | |
| A | CLINICAL IMMUNOLOGY AND IMMUNOPATHOLOGY vol. 50, 1989, pages 298 - 306; ERI MUSO ET AL: 'Specific Binding to Methylated Polynucleotides in Monoclonal Anti-poly(dT) Antibodies from Autoimmune Mice' <br> * abstract * * <br> – – – – – | 1 | |

**TECHNICAL FIELDS SEARCHED (Int. Cl.5)**

C 12 Q

The present search report has been drawn up for all claims

| Place of search | Date of completion of search | Examiner |
|---|---|---|
| The Hague | 25 October 91 | LUZZATTO E.R.P.G.A. |